# DEMANDE DE BREVET EUROPEEN

(11) **EP 0 994 154 A1**
(43) Date de publication de la demande: **19.04.2000**
(21) Numéro de dépôt: 99402381.0
(22) Date de dépôt: 29.09.1999
(51) Int. Cl.: C08L 57/00, C09J 157/04, A61K 7/06

(54) **Composition adhésive comprenant un polymère filmogène et une silicone oxyalkylènée**

(30) Priorité: 15.10.1998 FR 9812954
(71) Demandeur: L'OREAL, 75008 Paris (FR)
(72) Inventeur: Bodelin, Sophie, 92170 Vanves (FR); Piot, Bertrand, 75009 Paris (FR)
(74) Mandataire: Lhoste, Catherine

(57) **Abrégé**

L'invention a pour objet une composition adhésive cosmétique comprenant, dans un milieu aqueux, au moins un polymère radicalaire sous forme de particules dispersées dans le milieu aqueux, ledit polymère radicalaire étant apte à former un film ayant une dureté inférieure à 30 secondes, la composition comprenant au moins une silicone oxyalkylénée.

La composition est destinée au maintien d'articles d'embellissement sur les matières kératiniques comme la peau ou les ongles.

## Description

L'invention a pour objet une composition adhésive cosmétique comprenant un polymère filmogène et une silicone, destinée à être appliquée sur les matières kératiniques, notamment sur la peau ou les ongles d'être humain. La composition adhésive peut être employée pour coller des patchs, des disques, des pastilles, notamment de type mouche, des faux-cils ou bien encore des faux-ongles.

Les articles d'embellissement, comme les faux-cils, les faux-ongles, les pastilles de type mouche, sont généralement maintenus sur les matières kératiniques à l'aide de compositions adhésives adaptées à cette utilisation. Ces compositions, en plus de leurs propriétés adhésives, ne doivent pas être toxiques.

Parmi les compositions adhésives de l'art antérieur destinées à être appliquées sur la peau, il est connu des compositions comprenant des particules de polymères dispersées dans un milieu aqueux. Par exemple, le document SU-A-1351962 décrit une composition adhésive comprenant un copolymère acrylate de butyle/acide acrylique en dispersion aqueuse, employées pour le maintien des pansements. La demande JP-A-57-092073 décrit un adhésif pour fausses moustaches comprenant un latex de polyisoprène. La demande JP-A-52-100532 décrit un adhésif cosmétique pour les cils comprenant une émulsion aqueuse de copolymère d'esters vinyliques.

Or, les compositions adhésives à base de polymère dispersé en milieu aqueux ont l'inconvénient de laisser un résidu sur le support kératinique, comme par exemple la peau, lorsque l'on retire l'article d'embellissement collé dudit support. Ce résidu ne s'élimine pas facilement à l'aide de démaquillants classiques et son élimination nécessite un frottement important du support kératinique, engendrant une sensation d'inconfort, voire d'irritation de la peau, pour l'utilisateur.

Le but de la présente invention est de disposer d'une composition adhésive cosmétique, comprenant un polymère sous forme de particules dispersées dans un milieu aqueux, pouvant être appliquée sur les matières kératiniques comme la peau ou les ongles et facile à éliminer à l'eau chaude et au savon, ou bien encore à l'aide des démaquillants classiques.

Le demandeur a découvert qu'une telle composition pouvait être obtenue en employant, avec le polymère, une silicone particulière.

De façon plus précise, l'invention a pour objet une composition adhésive cosmétique comprenant, dans un milieu aqueux, au moins un polymère radicalaire sous forme de particules dispersées dans le milieu aqueux, caractérisée par le fait que ledit polymère radicalaire est apte à former un film ayant une dureté inférieure à 30 secondes, et que la composition comprend au moins une silicone oxyalkylénée.

L'invention a également pour objet l'utilisation de la composition telle que définie précédemment comme agent adhésif destiné à être appliqué sur les matières kératiniques, notamment sur la peau et les ongles.

L'invention a encore pour objet un procédé de collage d'article sur des matières kératiniques, consistant à déposer au moins une couche de composition adhésive telle que définie précédemment sur les matières kératiniques et/ou sur l'article et à mettre en contact l'article et les matières kératiniques.

La composition selon l'invention présente de bonnes propriétés adhésives sur les matières kératiniques comme la peau et les ongles, et s'élimine facilement, après séchage, à l'eau chaude et au savon, sans irriter la peau.

La composition selon l'invention comprend donc au moins un polymère filmogène synthétique de type radicalaire.
Par polymère radicalaire, on entend un polymère obtenu par polymérisation de monomères à insaturation notamment éthylénique, chaque monomère étant susceptible de s'homopolymériser (à l'inverse des polycondensats).

La dureté du film est mesurée pour une couche de 300 µm d'épaisseur (avant séchage), déposée sur une plaque de verre chauffée à 30 °C et après séchage, pendant 24 heures à 30 °C, à 50 % d'humidité relative. La dureté du film obtenu est mesurée selon la norme ASTM D-43-66, ou la norme NF-T 30-016 (décembre 1991), à l'aide d'un pendule de Persoz.

De préférence, le polymère filmogène radicalaire utilisé dans la composition selon l'invention peut être apte à former un film ayant une dureté inférieure ou égale à environ 20 secondes. De façon avantageuse, la dureté du film est supérieure à 1 seconde, et notamment supérieure à environ 5 secondes.
Préférentiellement, le film de polymère peut avoir une dureté allant environ de 5 à 20 secondes, et notamment de 13 à 18 secondes.

Dans la composition selon l'invention, les polymères de type radicalaires peuvent être notamment des polymères, ou des copolymères, vinyliques, notamment des polymères acryliques.

Les polymères vinyliques peuvent résulter de la polymérisation de monomères à insaturation éthylénique ayant au moins un groupement acide et/ou des esters de ces monomères acides et/ou des amides de ces monomères acides.

On utilise de préférence des polymères radicalaires anioniques, c'est-à-dire des monomères ayant au moins un monomère à groupement acide.

Comme monomère porteur de groupement acide, on peut utiliser des acides carboxyliques insaturés α,β-éthyléniques tels que l'acide acrylique, l'acide méthacrylique, l'acide crotonique, l'acide maléique, l'acide itaconique, ainsi que l'acide 2-acrylamide 2-méthyl propane sulfonique. On utilise de préférence l'acide (méth)acrylique et l'acide crotonique, et plus préférentiellement l'acide (méth)acrylique.

Les esters de monomères acides sont avantageusement choisis parmi les esters de l'acide (méth)acrylique (encore appelé les (méth)acrylates), notamment des (méth)acrylates d'alkyle, en particulier d'alkyle en C₁-C₂₀, de préférence en C₁-C₈, des (méth)acrylates d'aryle, en particulier d'aryle en C₆-C₁₀, des (méth)acrylates d'hydroxyalkyle, en particulier d'hydroxyalkyle en C₂-C₆.
Parmi les (méth)acrylates d'alkyle, on peut citer le méthacrylate de méthyle, le méthacrylate d'éthyle, le méthacrylate de butyle, le méthacrylate d'isobutyle, le méthacrylate d'éthyl-2 hexyle, le méthacrylate de lauryle.
Parmi les (méth)acrylates d'hydroxyalkyle, on peut citer l'acrylate d'hydroxyéthyle, l'acrylate de 2-hydroxypropyle, le méthacrylate d'hydroxyéthyle, le méthacrylate de 2-hydroxypropyle.
Parmi les (méth)acrylates d'aryle, on peut citer l'acrylate de benzyle et l'acrylate de phényle.
Les esters de l'acide (méth)acrylique particulièrement préférés sont les (méth)acrylates d'alkyle.

Selon la présente invention, le groupement alkyle des esters peut être soit fluoré, soit perfluoré, c'est-à-dire qu'une partie ou la totalité des atomes d'hydrogène du groupement alkyle sont substitués par des atomes de fluor.

Comme amides des monomères acides, on peut par exemple citer les (méth)acrylamides, et notamment les N-alkyl (méth)acrylamides, en particulier d'alkyl en C₂-C₁₂. Parmi les N-alkyl (méth)acrylamides, on peut citer le N-éthyl acrylamide, le N-t-butyl acrylamide et le N-t-octyl acrylamide.

Les polymères vinyliques peuvent également résulter de l'homopolymérisation ou de la copolymérisation de monomères choisis parmi les esters vinyliques et les monomères styrèniques. En particulier, ces monomères peuvent être polymérisés avec des monomères acides et/ou leurs esters et/ou leurs amides, tels que ceux mentionnés précédemment.
Comme exemple d'esters vinyliques, on peut citer l'acétate de vinyle, le néodécanoate de vinyle, le pivalate de vinyle, le benzoate de vinyle et le t-butyl benzoate de vinyle.
Comme monomères styrèniques, on peut citer le styrène et l'alpha-méthyl styrène.

La liste des monomères donnée n'est pas limitative et il est possible d'utiliser tout monomère connu de l'homme du métier dans les catégories de monomères acryliques et vinyliques.

Selon l'invention, on utilise de préférence comme polymère filmogène un copolymère choisi parmi les copolymères acide (méth)acrylique / (méth)acrylate, acide (méth)acrylique / (méth)acrylate / styrène, acide (méth)acrylique/ styrène, acide (méth)acrylique / α-méthyl styrène ainsi que les copolymères de (méth)acrylates. Préférentiellement, on utilise un copolymère issu de la copolymérisation de monomères méthacrylates d'alkyle en C₁-C₈, éventuellement associés à l'acide acrylique, au styrène et à l'α-méthyl styrène.

Lorsque le polymère utilisé selon l'invention comprend des monomères porteur de groupement salifiable (par exemple un groupe acide carboxylique), il peut être neutralisé, totalement ou en partie à l'aide d'un agent neutralisant (en l'occurrence une base pour neutraliser le groupement acide) bien connu de l'homme du métier. La neutralisation peut, en outre, favoriser la mise en dispersion, notamment dans l'eau, du polymère, voire même stabiliser ladite dispersion.

La dispersion aqueuse comprenant un ou plusieurs polymères filmogènes peut être préparée par l'homme du métier sur base de ses connaissances générales.

La taille des particules de polymères en dispersion aqueuse peut aller de 10 à 500 nm, et de préférence de 20 à 150 nm.

Comme dispersion aqueuse de polymère utilisable selon l'invention, on peut par exemple citer celles vendues sous les dénominations "DOW LATEX 432^{®}" (dureté = 6,8) par la société Dow Corning, "CARBOSET 515^{®}" (dureté = 14,7) par la société GOODRICH, "NEOCRYL A-1070^{®}" et "NEOCRYL A-1090^{®}" (dureté = 15,4), "NEOCRYL A-523^{®}" (dureté = 19,8) par la société ZENECA, "DYFLEX LP 9619^{®}" (dureté = 25,7) par la société DYFLEX.

Dans la composition selon l'invention, le polymère filmogène radicalaire peut être présent en une teneur allant de 20 à 50 % en poids, par rapport au poids total de la composition, et de préférence de 30 à 45 % en poids.

La silicone oxyalkylénée de la composition selon l'invention peut être choisie parmi les diméthicone copolyols.

Les diméthicone copolyols peuvent être choisis parmi les composés de formule générale (I) : 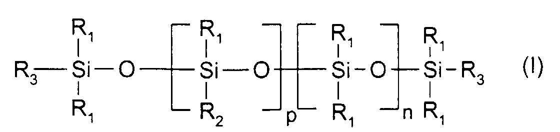 formule dans laquelle :
- R₁, identiques ou différents, représentent un atome d'hydrogène, un radical alkyle linéaire ou ramifié en C₁-C₃₀ ou un radical phényle,
- R₂, identique ou différent, représente R₁ ou A = -(CₓH₂ₓ)-(OC₂H₄)ₐ-(OC₃H₆)_{b}-OR₄,
- R₃, identiques ou différents, désignent R₁ ou A, avec R₂ différent de R₃ quand R₂ = A ou R₃ = A,
- R₄, identiques ou différents, sont choisis parmi un atome d'hydrogène, un radical alkyle, linéaire ou ramifié, ayant de 1 à 12 atomes de carbone, un radical acyle, linéaire ou ramifié ayant de 2 à 6 atomes de carbone,
- n varie de 0 à 1000,
- p varie de 1 à 50,
- a varie de 0 à 50,
- b varie de 0 à 50,
- a + b est supérieur ou égal à 1,
- x varie de 1 à 5,
- le poids moléculaire moyen en nombre étant supérieur ou égal à 900 et de préférence compris entre 2000 et 75000.

De façon préférentielle, on utilise les silicones oxyalkylénées de formule générale (I) qui répondent à au moins une des, et de préférence à toutes les, conditions suivantes :
- R₁ désigne le radical méthyle,
- R₂ = A,
- R₃ = R₁,
- R₄ représente un atome d'hydrogène, un radical méthyle ou un radical acétyle, et de préférence hydrogène,
- p varie de 8 à 20,
- a est compris entre 5 et 40 et de préférence entre 15 et 30,
- b est compris entre 5 et 40 et de préférence entre 15 et 30,
- x est égal à 2 ou 3,
- n varie de 20 à 600, de préférence de 50 à 500 et encore plus particulièrement de 100 à 300.
De telles silicones sont par exemple décrites dans le brevet US-4,311,695 qui est inclus à titre de référence.

Des diméthicones copolyols ont en particulier été présentés par la société DOW CORNING lors du 17ème congrès international de l'I.F.S.C.C. d'octobre 1992 et rapportés dans l'article "Water-soluble dimethicone copolyol waxes for personal care industry" de Linda Madore et al., pages 1 à 3. On peut également utiliser celles décrites dans la demande EP-A-331833 dont le contenu est inclus dans la présente demande à titre de référence.
Ces diméthicones copolyols sont des polydiméthylsiloxanes (PDMS) comportant une ou plusieurs fonctions éthers, solubles dans l'eau (oxyalkylène, notamment oxyéthylène et/ou oxypropylène).
De tels diméthicones copolyols sont notamment vendus par la société GOLDSCHMIDT sous la dénomination ABIL B8851, ABIL B88183, ABIL WE09, ABIL EM90, ABIL EM97. On peut citer aussi les composés KF 351 à 354 et KF 615 A vendus par la société SHIN ETSU ou la DMC 6038 de la société WACKER.
Les dérivés de diméthicones copolyols utilisables peuvent être en particulier les diméthicones copolyols à groupement phosphate, sulfate, chlorure de myristamide propyldiméthylammonium, stéarate, amine, glycomodifié, etc. On peut utiliser comme dérivés de diméthicones copolyols notamment les composés vendus par la société SILTECH sous la dénomination Silphos A100, Siltech amine 65, Silwax WDIS, myristamido silicone quat, ou par la société PHOENIX sous la dénomination Pecosil PS 100.
On peut également utiliser les dérivés vendus par la société WACKER sous la dénomination VP 1661, ou par la société DOW CORNING sous la dénomination 2501 cosmetic wax. On peut encore utiliser les dérivés vendus par la société WACKER sous la dénomination VP 1661, ou par la société DOW CORNING sous la dénomination 2501 cosmetic wax.
Les silicones les plus particulièrement préférées sont par exemple celles vendues par la société DOW CORNING sous la dénomination commerciale Q2-5220 et par la société RHONE POULENC sous la dénomination MIRASIL DMCO.

Dans la composition selon l'invention, la silicone oxyalkylénée peut être présente en une teneur allant de 2 à 10 % en poids, par rapport au poids total de la composition, et de préférence de 3 à 8 % en poids.

Pour favoriser le séchage rapide de la composition sur la peau et/ou les ongles, la composition peut comprendre des accélérateurs de séchage comme les solvants volatils et de préférence des solvants organiques volatils miscibles à l'eau, comme l'éthanol. Ces solvants organiques peuvent être présent dans la composition en une teneur allant jusqu'à 20 % en poids, par rapport au poids total de la composition, (notamment de 0,1 % à 20 %) et de préférence de 10 % à 20 % en poids. Par volatil, on entend un composé apte à s'évaporer au contact de la peau, à température ambiante.

L'invention peut comprendre, en outre, d'autres additifs cosmétiques dont l'emploi ne doit pas altérer les propriétés adhésives de la composition. De tels additifs peuvent être choisis, de façon connue, parmi les conservateurs, les colorants, les pigments, les charges, les humectants, les vitamines, les agents neutralisants, les épaississants.

L'invention est illustrée plus en détail dans les exemples suivants.

### Exemple 1:

On a préparé une colle cosmétique ayant la composition suivante :
- polymère acrylate en dispersion aqueuse à 45 % en poids de matières sèches (NEOCRYL A-1090 de ZENECA) 36 g MA
- diméthicone copolyol vendu sous la dénomination " Q2-5220 " par la société Dow Corning 5 g
- alcool éthylique 15 g
- eau qsp 100 g

La composition adhère bien sur la peau, sèche rapidement et permet de fixer sur la peau des pastilles d'embellissement de type mouche. En outre, la colle s'enlève très facilement à l'eau tiède savonneuse.

### Exemple 2 :

On a préparé une colle cosmétique ayant la composition suivante :
- polymère acrylate en dispersion aqueuse à 45 % en poids de matières sèches (NEOCRYL A-1090 de ZENECA) 38,2 g MA
- diméthicone copolyol vendu sous la dénomination " Q2-5220 " par la société Dow Corning 5 g
- alcool éthylique 10 g
- eau qsp 100 g

La composition adhère bien sur la peau, sèche rapidement et permet de fixer sur la peau des articles d'embellissement de type mouche. En outre, la colle s'enlève très facilement à l'eau tiède savonneuse.

## Revendications

1. Composition adhésive cosmétique comprenant, dans un milieu aqueux, au moins un polymère radicalaire sous forme de particules dispersées dans le milieu aqueux, caractérisée par le fait que ledit polymère radicalaire est apte à former un film ayant une dureté inférieure à 30 secondes, et que la composition comprend au moins une silicone oxyalkylénée.

2. Composition selon l'une quelconque des revendications précédentes, caractérisée par le fait que la dureté du film est inférieure ou égale à 20 secondes.

3. Composition selon l'une quelconque des revendications précédentes, caractérisée par le fait que la dureté du film va de 5 à 20 secondes, et mieux de 13 à 18 secondes.

4. Composition selon l'une quelconque des revendications précédentes, caractérisée par le fait que le polymère radicalaire est un polymère issu d'au moins un monomère éthylénique.

5. Composition selon l'une quelconque des revendications précédentes, caractérisée par le fait que le polymère radicalaire résulte de la polymérisation de monomères choisis dans le groupe formé par les acides carboxyliques insaturés α,β-éthyléniques, les (méth)acrylates, les (méth)acrylamides, les esters vinyliques, les monomères styréniques.

6. Composition selon l'une quelconque des revendications précédentes, caractérisée par le fait que le polymère radicalaire est choisi dans le groupe des copolymères formé par les copolymères acide (méth)acrylique / (méth)acrylate, acide (méth)acrylique / (méth)acrylate / styrène, acide (méth)acrylique/ styrène, acide (méth)acrylique/α-méthyl styrène, les copolymères de (méth)acrylates d'alkyles.

7. Composition selon l'une des revendications 5 ou 6, caractérisée par le fait que les (méth)acrylates sont choisis parmi les (méth)acrylates d'alkyle, de préférence d'alkyle en C₁-C₂₀.

8. Composition selon la revendication 7, caractérisée par le fait que le polymère radicalaire est un copolymère issu de la copolymérisation de monomères méthacrylates d'alkyle en C₁-C₈, éventuellement associés à l'acide acrylique, au styrène et à l'α-méthyl styrène.

9. Composition selon l'une quelconque des revendications précédentes, caractérisée par le fait que les particules de polymère ont une taille allant de 10 à 500 nm, et de préférence de 20 à 150 nm.

10. Composition selon l'une quelconque des revendications précédentes, caractérisée par le fait qu'elle comprend une teneur en polymère filmogène radicalaire allant de 20 à 50 % en poids de matière sèche, de préférence de 30 à 45% en poids, par rapport au poids total de la composition.

11. Composition selon l'une quelconque des revendications précédentes, caractérisée en ce que la silicone oxyalkylénée est choisie parmi les composés de formule générale (I): 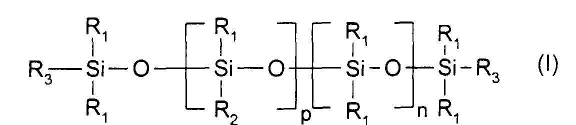 dans laquelle :
- R₁, identiques ou différents, représentent un atome d'hydrogène, un radical alkyle linéaire ou ramifié en C₁-C₃₀ ou un radical phényle,
- R₂, identique ou différent, représente R₁ ou A = -(CₓH₂ₓ)-(OC₂H₄)ₐ-(OC₃H₆)_{b}-OR₄,
- R₃, identiques ou différents, désignent R₁ ou A, avec R₂ différent de R₃ quand R₂ = A ou R₃ = A,
- R₄, identiques ou différents, sont choisis parmi un atome d'hydrogène, un radical alkyle, linéaire ou ramifié, ayant de 1 à 12 atomes de carbone, un radical acyle, linéaire ou ramifié ayant de 2 à 6 atomes de carbone,
- n varie de 0 à 1000,
- p varie de 1 à 50,
- a varie de 0 à 50,
- b varie de 0 à 50,
- a + b est supérieur ou égal à 1,
- x varie de 1 à 5,
le poids moléculaire moyen en nombre étant supérieur ou égal à 900 et de préférence compris entre 2000 et 75000.

12. Composition selon la revendication 11, caractérisée en ce qu'elle comprend une silicone oxyalkylénée de formule générale (I) qui répondent à au moins une des, et de préférence à toutes les, conditions suivantes :
- R₁ désigne le radical méthyle,
- R₂ = A,
- R₃ = R₁,
- R₄ représente un atome d'hydrogène, un radical méthyle ou un radical acétyle, et de préférence hydrogène,
- p varie de 8 à 20.
- a est compris entre 5 et 40 et de préférence entre 15 et 30.
- b est compris entre 5 et 40 et de préférence entre 15 et 30.
- x est égal à 2 ou 3.
- n varie de 20 à 600, de préférence de 50 à 500 et encore plus particulièrement de 100 à 300.

13. Composition selon l'une quelconque des revendications précédentes, caractérisée par le fait que la silicone oxyalkylénée est présente en une teneur allant de 2 à 10 % en poids, de préférence de 3 à 8 % en poids, par rapport au poids total de la composition.

14. Composition selon l'une quelconque des revendications précédentes, caractérisée par le fait qu'elle comprend, en outre, au moins un accélérateur de séchage.

15. Composition selon la revendication 14, caractérisée par le fait que l'accélérateur de séchage est un solvant organique miscible à l'eau.

16. Composition selon la revendication 14 ou 15, caractérisée par le fait que l'accélérateur de séchage est de l'éthanol.

17. Composition selon l'une quelconque des revendications 14 à 16, caractérisée par le fait que l'accélérateur de séchage est présent en une teneur allant de 0,1 % à 20 % en poids, et de préférence de 10 % à 20 % en poids, par rapport au poids total de la composition.

18. Utilisation d'une composition selon l'une quelconque des revendications 1 à 17 comme agent adhésif sur la peau ou les ongles.

19. Procédé de collage d'article sur des matières kératiniques, consistant à déposer au moins une couche de composition adhésive sur les matières kératiniques et/ou sur l'article et à mettre en contact l'article et les matières kératiniques, caractérisé par le fait que la composition adhésive est une composition selon l'une quelconque des revendications 1 à 17.
